# EUROPEAN PATENT APPLICATION

(11) **EP 0 742 204 A1**
(43) Date of publication of application: **13.11.1996**
(21) Application number: 96105991.2
(22) Date of filing: 17.04.1996
(51) Int. Cl.: C07C 403/20

(54) **Photochemical method for the preparation of 13-cis-retinoic acid**

(30) Priority: 12.05.1995 IT MI950973
(71) Applicant: LABORATORI MAG S.p.A., 20131 Milano (IT)
(72) Inventor: Magnone, Angelo Grato, 20131 Milan (IT)
(74) Representative: Gervasi, Gemma, Dr.

(57) **Abstract**

Photochemical isomerization of 11-cis,13-cis-retinoic acid to 13-cis-retinoic acid.

## Description

### Field of the invention

The present invention relates to the photochemical isomerization of 11-cis,13-cis-retinoic acid to 13-cis-retinoic acid.

### Prior art

13-cis-retinoic acid of formula (I) 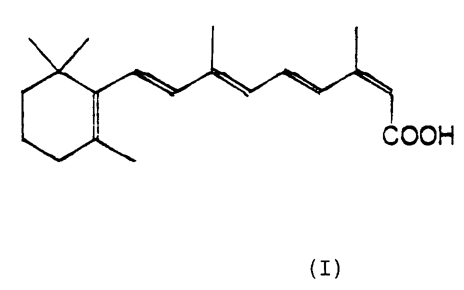 is an active ingredient that finds wide practical application as a drug, in particular for the treatment of acne.

A process for the preparation of this product is disclosed in "Carotenoids and Related Compounds, Part XVIII, Synthesis of cis and di-cis-polyenes by reactions of Wittig type", Paddenten G. and Weedon B.C.L., J. Chem. Soc., (C), 1984-1997, 1968. Said process comprises a Wittig condensation at room temperature in an ether solvent in the presence of sodium methoxide between [3-methyl-5-(2,6,6-trimethyl-1-cyclohexen-1-yl-2,4-pentadienyl]-triphenylphosphonium halide of formula (II) 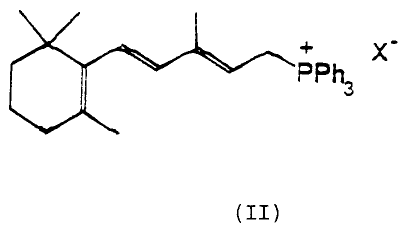 where X is a halogen atom,
and 5-hydroxy-4-methyl-2(5-H)-furanone of formula (III)

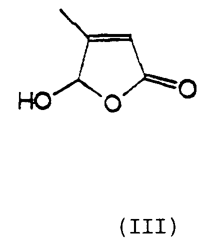

However, a disadvantage of this process is that the desired product is obtained in a mixture with the following isomers, i.e. 11-cis,13-cis-retinoic acid of formula (IV) 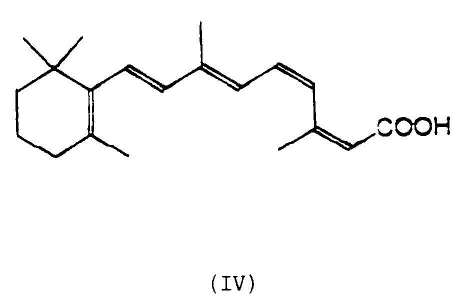 and 11-trans,13-trans-retinoic acid (i.e. vitamin A acid) of formula (V)

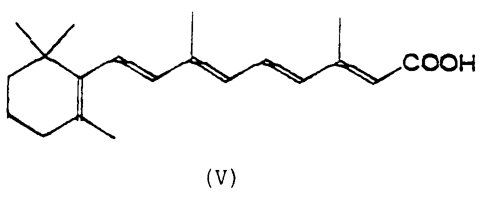

Therefore, product (I), which is obtained in smaller amounts in comparison with isomer (IV) and in amounts more or less comparable to isomer (V), has to be separated from the mixture by conventional methods, i.e. silica gel chromatography or fractional crystallization, bringing about a further yield decrease.

EP 0111325 discloses a process for the preparation of 13-cis-retinoic acid, wherein the Wittig condensation of product (II) with product (III) takes place at low temperatures (from -10° to -50°C) in an alcoholic solvent and in the presence of bases consisting of alkaline metal hydroxides, e.g. potassium hydroxide.

The conversion obtained is above 90% with respect to the starting product (III), the reaction product consists of a mixture containing from 10% to 30% of isomer (I) and from 70% to 90% of isomer (IV).

The mixture as such or isomer (IV) alone, previously separated from the aforesaid mixture, are subjected to isomerization in the presence of catalysts based on rhodium or palladium, whereby product (IV) is converted into 13-cis-retinoic acid.

This process gives 13-cis-retinoic acid in higher yields than the Paddenten and Weedon's process. However, a disadvantage of said process is that the isomerization of 11-cis,13-cis-retinoic acid of formula (IV) takes place in the presence of catalysts based on extremely costly transition metals, such as rhodium and palladium. Furthermore, it is very difficult to recover chemically pure 13-cis-retinoic acid as prescribed by the manufacturing regulations in force from the reaction raw products containing said catalysts.

It follows that there is a need for providing a simple isomerization allowing to increase the 13-cis-retinoic acid content in the isomer mixture obtained by Wittig condensation, without the inconveniences inherent in the isomerization described in EP111325, and for recovering a highly pure product by simple and conventional methods.

### Summary

The Applicant has surprisingly found that 11-cis,13-cis-retinoic acid of formula (IV) may be converted into 13-cis-retinoic acid by irradiation in the visible spectrum at room temperature, in an apolar aprotic solvent, in the presence of photoactivators.

The photochemical isomerization efficiency in converting all cis double bonds into trans double bonds of polyunsaturated systems, e.g. for the preparation of vitamin A, is disclosed in DE 2210800 and CH 605724.

It was, therefore, wholly unexpected that the photochemical activation, at suitable operating conditions, might mainly convert the cis double bond in position 11 and not the trisubstituted cis double bond in position 13 of 11-cis,13-cis-retinoic acid (IV) into a trans double bond.

In fact, by said operation, the 13-cis-retinoic acid forms from product (IV) much more rapidly than the entirely trans isomer (V).

### Detailed description of the invention

The photochemical isomerization of 11-cis, 13-cis-retinoic acid to 13-cis-retinoic acid was carried out by irradiation in the visible spectrum extending from a wavelength of 4000 Å to 7000 Å. The choice of the photoactivator has no significant effect; however, Bengal rose, diphenyldisulphide. cercosporin, and chlorophyll are preferably used.

The photochemical isomerization is preferably conducted in apolar aprotic solvents, e.g. methylene chloride, acetonitrile 1,2-dichloroethane or mixtures thereof.

The photochemical isomerization according to the present invention is preferably used to increase the product (I) content in the mixtures derived from Wittig's condensation between the aforementioned compounds (II) and (III) (cf. "Carotenoids and Related Compounds, Part XVIII, Synthesis of cis and di-cis-polyenes by reactions of Wittig type", Paddenten G. and Weedon B.C.L., J. Chem. Soc., (C), 1984-1997, 1968) at room temperature, in an ether solvent, in the presence of sodium methoxide.

The Applicant has also found that when the photochemical isomerization is carried out according to the present invention and, in particular, using Bengal rose on mixtures derived from said condensation, the 13-cis-retinoic acid may be obtained in 75% yields with respect to the total, when the aforesaid starting mixtures contained it in smaller amounts in comparison with isomer (IV) and in amounts more or less comparable to isomer (V). From a so 13-cis-retinoic acid-rich mixture, the product may be easily separated by fractional crystallization with 50% yields, which are fully comparable with those obtained by isomerization in the presence of catalysts based on rhodium and palladium. A further amount of 13-cis-retinoic acid may be recovered from the crystallization mother liquors, previously treated for photoactivator removal.

Product (I) derived from fractional crystallization may optionally be subjected to a further recrystallization in an alcoholic solvent, preferably isopropanol.

The isomerization according to the present invention may also be used to isomerize mixtures derived from Wittig's condensation carried out at low temperatures in an alcoholic solvent in the presence of alkaline metal hydroxides, as disclosed in the aforementioned EP 0111325.

The following examples are conveyed by way of indication, not of limitation, of the isomerization process according to the present invention.

### EXAMPLE 1

### Preparation of the mixture of retinoic acids (I), (IV) and (V)

80g triphenylphosphine bromhydrate and 50 vinyl β-Ionol were added to 300 ml methanol. The resulting mixture was stirred overnight, the solvent was evaporated, and the reaction raw product was taken up with 500 ml anhydrous ether. The reaction mixture was treated with 50 ml sodium methylate, obtained from 6.9 g metal sodium, and, 20 minutes after, 8 g butenolide were added to 10 ml ether. One hour later, the mixture was diluted with 200 ml hexane and extracted three times with 100 ml 1:1 methanol-water mixture. The mixture of retinoic acids was extracted three times with 100 ml ether from acidified methanolic water. The ether phase was washed with a 3:1 methanol-water mixture, then with water and sodium chloride. Water was eliminated from the organic phase and the solvent was evaporated. HPLC analysis revealed that the mixture obtained (33 g) consisted of:
13-cis-retinoic acid (I): 23%
11-cis,13-cis retinoic acid (IV): 64%
11-trans,13-trans-retinoic acid (V): 13%

### EXAMPLE 2

3 g of the mixture prepared as described in Example 1 were dissolved in a 3:7 methylene-acetonitrile mixture and fed to a glass reactor, externally provided with a series of 8 Watt Hitachi lamps, F8T5/CW, placed in a Rayonet type apparatus. The reactor was then fed with diphenyldisulphide (starting mixture-diphenylsulphide molar ratio of 10:1). The reaction mixture was subjected to 20 hrs' irradiation and analysed by HPLC.

HPLC analysis gave the following results: (I) 53%, (IV) 4%, (V) 43%.

### EXAMPLE 3

20 g of the mixture prepared as described in Example 1 were dissolved in a 3:7 methylene-acetonitrile mixture and fed to the reactor described in Example 2. The reactor was then fed with 700 mg Bengal rose (starting mixture-Bengal rose molar ratio of 100:1). The reaction mixture was subjected to 40 hrs irradiation and analysed by HPLC.

HPLC analysis gave the following results: (I) 74.5%, (IV) 0.89%, (V) 24.6%.

### EXAMPLE 4

The test was carried out according to the method of Example 3.

6.1 g precipitate, separated during the photochemical treatment, consisting of 97.5% of product (I), were collected. A further precipitate in quantities of 3.7 g, obtained by mother liquors concentration, was dissolved in 15 ml methylene chloride. The resulting solution was filtered on a silica column for Bengal rose removal. 2 g of a product consisting of 91.6% of product (I) and, successively, 1 g of a product consisting of 82% of product (I) were obtained.

### EXAMPLE 5

The test was carried out according to the method of Example 3.

11 g precipitate, consisting of 86% of isomer (I), were collected. Recrystallization of this product from isopropanol gave 8.5 g of pure (I). Yield: 42.5%.

## Claims

1. Process for the preparation of 13-cis-retinoic acid (I) 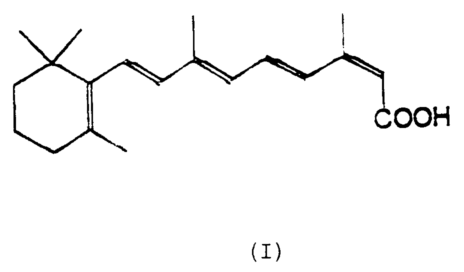 by light irradiation in the visible spectrum of 11-cis,13-cis-retinoic acid (IV), at room temperature, in an apolar aprotic solvent, in the presence of a photoactivator selected among Bengal rose, diphenyldisulphide, cercosporin, and chlorophyll.

2. The process according to claim 1, wherein the said apolar aprotic solvent is selected among methylene, acetonitrile 1,2 dichloroethane or mixtures thereof.

3. The process according to claim 1, utilized to increase the product (I) content in the mixtures also containing products (I) and (V), coming from Wittig's condensation between [3-methyl-5-(2,6,6-trimethyl-1-cyclohexen-1-yl-2,4-pentadienyl]-triphenylphosphonium halide of formula (II) 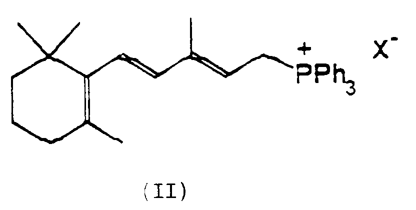 where X is a halogen atom,
and 5-hydroxy-4-methyl-2(5-H)-furanone of formula (III) 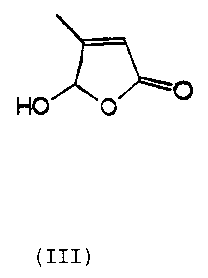 is carried out at room temperature in an ether solvent and in the presence of sodium methoxide.

4. The process according to claim 3, wherein the said product (I) obtained by light irradiation is separated by fractional crystallization and a further amount of same is recovered from the crystallization mother liquors.

5. The process according to claim 1, wherein the said process used with mixtures mainly containing isomers (IV) and (I), derived from Wittig's condensation between [3-methyl-5-(2,6,6-trimethyl-1-cyclohexen-1-yl-2,4-pentadienyl]-triphenylphosphonium halide of formula (II) 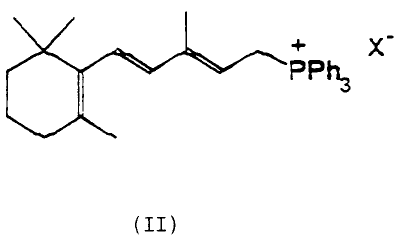 where X is a halogen atom,
and 5-hydroxy-4-methyl-2(5-H)-furanone of formula (III) 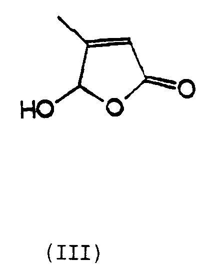 is carried out at a temperature ranging from -10° to -50°C in an alcoholic solvent and in the presence of bases consisting of alkaline metal hydroxides.
